(19) Europäisches Patentamt / European Patent Office / Office européen des brevets

(11) **EP 4 744 679 A1**

(12) **EUROPEAN PATENT APPLICATION**
published in accordance with Art. 153(4) EPC

(43) Date of publication:
**20.05.2026 Bulletin 2026/21**

(21) Application number: **24838683.1**

(22) Date of filing: **04.07.2024**

(51) International Patent Classification (IPC):
**A61K 47/34** (2017.01)   **A61L 17/10** (2006.01)
**A61L 27/58** (2006.01)   **A61P 41/00** (2006.01)

(52) Cooperative Patent Classification (CPC):
**A61K 31/192; A61K 31/4152; A61K 31/444;
A61K 31/635; A61K 31/765; A61K 45/00;
A61K 47/34; A61L 17/10; A61L 27/58; A61P 19/04;
A61P 29/00; A61P 41/00**

(86) International application number:
**PCT/CN2024/103598**

(87) International publication number:
**WO 2025/011430 (16.01.2025 Gazette 2025/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.07.2023  CN 202310840075**

(71) Applicant: **Changchun Sinobiomaterials Co., Ltd.
Changchun, Jilin 130000 (CN)**

(72) Inventors:
• **WANG, Jinyue**
  Changchun, Jilin 130000 (CN)
• **ZHUANG, Xiuli**
  Changchun, Jilin 130000 (CN)
• **ZHANG, Tianhui**
  Changchun, Jilin 130000 (CN)

(74) Representative: **Colombo, Stefano Paolo et al
Marchi & Partners S.r.l.
Via Vittor Pisani, 13
20124 Milano (IT)**

(54) **USE OF POLYLACTIC ACID AND COPOLYMER THEREOF IN PREPARATION OF DRUGS FOR PROMOTING TENDON/LIGAMENT GROWTH**

(57)    Provided is a use of polylactic acid and a copolymer thereof in preparation of drugs for promoting tendon/ligament growth. The molecular weight of the polylactic acid is 400-300000 Da, and preferably 5000-20000 Da. The polylactic acid copolymer comprises one or more of a poly(lactic acid-glycolic acid) copolymer, a poly(lactic acid-ethylene glycol) copolymer, a poly(ethylene glycol-lactic acid-glycolic acid) copolymer, a polylactic acid-chitosan copolymer, and a lactide-caprolactone copolymer, preferably a poly(lactic acid-glycolic acid) copolymer. It is found for the first time that the polylactic acid and the copolymer thereof have the effect of promoting ligament/tendon growth, and a new approach is provided for treatment of ligament/tendon injury.

FIG. 3

**Description**

**CROSS-REFERENCE TO RELATED APPLICATIONS**

The present application claims priority to Chinese patent application No. 202310840075.X, filed on 10 July 2023 and entitled "Use of Polylactic Acid and Copolymer Thereof in Preparation of Drugs for Promoting Tendon/Ligament Growth", which is incorporated herein by reference in its entirety.

**TECHNICAL FIELD**

The present invention relates to the technical field of polymer drugs, and particularly to the use of polylactic acid and a copolymer thereof in the preparation of drugs for promoting tendon/ligament growth.

**BACKGROUND**

Joint ligament injuries are among the most common sports injuries, with a large number of new cases occurring worldwide each year, making it a global health problem. Tendons/ligaments are fibrous connective tissues that connect muscles to bones or bones to bones, respectively, for traction and stabilization. Once an injury occurs, it will significantly reduce the patient's quality of life, and in severe cases, it may lead to functional disability.

To restore knee joint function after ligament injury, ACL reconstruction surgery is often required. Currently, there are three types of clinical reconstruction grafts: autologous tendons, allogeneic tendons, and artificial ligaments. Among them, autologous tendons are taken from the patient's own hamstrings, gracilis, etc., and autologous tendon transplantation can cause secondary damage to the patient and is not conducive to later recovery. At the same time, the length and diameter of autologous tendons are limited by the harvesting site, making it difficult to meet the standards for use. Allogeneic tendons have the risk of immune rejection and infection in clinical use, and the source of donors is limited. Artificial ligaments are usually made of high molecular organic materials, and their degradation products can change the local microenvironment of the joint, which can easily cause aseptic inflammation. In addition, such tissues as tendons and cartilage lack blood supply, making tissue regeneration difficult. Grafts are mainly for functional replacement and do not have the ability to promote tissue regeneration. Therefore, biointegration of tissues with grafts is difficult, and there is still a risk of re-fracture after long-term implantation.

Polylactic acid (PLA) has the molecular formula (C3H4O2)n and is mainly prepared by lactic acid polymerization or lactide ring-opening polymerization. PLA and copolymers thereof not only possess excellent mechanical strength and chemical stability, but also exhibit good biocompatibility and biodegradability. In recent years, a great deal of research has been conducted both domestically and internationally on their application in biomedicine. It has been widely used in surgical sutures, bone repair materials, controlled-release drug systems, and tissue functional scaffolds (such as artificial bone and artificial skin).

Biodegradable and biocompatible poly-L-lactic acid microparticles (trade name SculptraTM) were approved by the U.S. FDA in 2004 for use in filling sagging skin areas caused by facial fat atrophy in AIDS patients. At the same time, clinicians also use it for age-related lipoatrophy and localized lipoatrophy in healthy individuals. In 2009, the U.S. FDA officially approved poly-L-lactic acid (PLA) fillers for use in improving nasolabial folds. However, there are no reports in the prior art regarding the role of polylactic acid and copolymers thereof in the repair of ligaments or tendons.

**SUMMARY OF THE INVENTION**

Aiming at the shortcomings existing in the prior art, the present invention provides the use of polylactic acid and copolymers thereof in the preparation of drugs for promoting tendon/ligament growth. It provides a new approach to promote tendon/ligament growth.

An objective of the present invention is to provide the use of polylactic acid and copolymers thereof in the preparation of drugs that promote tendon/ligament growth, wherein the polylactic acid has a molecular weight of 400-300,000 Da, preferably 5,000-20,000 Da.

Furthermore, the polylactic acid is poly-L-lactic acid.

Furthermore, the polylactic acid copolymer comprises one or more of a poly(lactic acid-glycolic acid) copolymer, a poly(lactic acid-ethylene glycol) copolymer, a poly(ethylene glycol-lactic acid-glycolic acid) copolymer, a polylactic acid-chitosan copolymer, and a lactide-caprolactone copolymer.

Preferably, the polylactic acid copolymer is a poly(lactic acid-glycolic acid) copolymer.

Furthermore, the polylactic acid and copolymers thereof exist in the form of a formulation.

Furthermore, the formulation includes microspheres, micelles, and gels, preferably microspheres. The preparation process of microspheres is simple and can achieve mass production; moreover, microspheres are easy to store and do not easily deteriorate.

Furthermore, the particle size range of the microspheres is 17 μm to 60 μm, preferably the particle size range of poly-L-lactic acid microspheres is 24 μm to 55 μm, and preferably the particle size range of poly(lactic acid-glycolic acid) microspheres is 17 μm to 56 μm. Microspheres within this particle size range can be injected with needles of 26G or larger, causing no invasive damage to the treatment site without the risk of the microspheres being too small. Microspheres with this particle size range degrade within 1 to 2 years, better meeting clinical needs. It also reduces the risk of microspheres entering blood vessels due to their too small size.

The formulation further comprises an excipient, and the excipient includes at least one of a stabilizer, a filler, a binder, a surfactant and a lubricant.

Furthermore, the surfactant includes one or more of polyethylene glycol, sodium dodecyl sulfonate, Tween, and Span; the stabilizer includes one or two of carboxymethyl cellulose and mannitol; the filler includes one or more of lactose, mannitol, cyclodextrin, and sorbitol; the binder includes one or more of hydroxypropyl cellulose, methyl cellulose, and polyvinylpyrrolidone; and the lubricant includes one or more of magnesium stearate, calcium stearate, and stearic acid.

Furthermore, the formulation further comprises a first active ingredient, and the first active ingredient has the effect of reducing swelling and relieving pain.

Furthermore, the first active ingredient includes celecoxib, loxoprofen sodium, phenylbutazone, ibuprofen, and etoricoxib.

Furthermore, during at least one administration of the formulation, the dosage of L-lactic acid in poly-L-lactic acid and copolymers thereof is 8-3000 mmol/L, preferably 250-750 mmol/L.

In a specific embodiment of the present invention, the formulation is injected into the diseased tendon, and the dose of the formulation is calculated according to the mass of the tendon at the diseased site.

In some embodiments, the above-mentioned formulation is an injection, and the dosage of poly-L-lactic acid or copolymers thereof based on L-lactic acid in the injection is 8-3000 mmol/L, preferably 250-750 mmol/L or 50-100 mmol/L, and for example, preferably 50-90 mmol/L, 50-80 mmol/L or 50-70 mmol/L. The above-mentioned injections include, but are not limited to, liquid injections and powder injections. When it is a powder injection, the above dosage refers to the dosage after the dry powder is reconstituted.

Compared with the prior art, the present invention has the following beneficial effects: The present invention is the first to discover that polylactic acid and copolymers thereof have the effect of promoting tendon/ligament growth, providing a new approach for the treatment of tendon/ligament injuries.

## BRIEF DESCRIPTION OF THE DRAWINGS

Fig. 1 is an SEM image of the PLLA microspheres in Example 1 of the present invention.

Fig. 2 is an SEM image of the PLGA microspheres in Example 2 of the present invention.

Fig. 3 shows the proliferation results of tendon cells under different concentrations of the PLLA microspheres in Example 3 of the present invention.

Fig. 4 shows the staining of the tendon on the experimental side (0.5 mg PLLA microspheres) and the control side (normal saline) in Example 4 of the present invention; red indicates collagen fibers.

Fig. 5 shows the staining of the tendon on the experimental side (0.5 mg PLGA microspheres) and the control side (normal saline) in Example 4 of the present invention; red indicates collagen fibers.

## DETAILED DESCRIPTION

The technical solution of the present invention will be further described in detail below in conjunction with specific embodiments. It should be understood that the following embodiments are only intended to exemplify and explain the present invention and should not be construed as limiting the scope of protection of the present invention. All technologies realized based on the above contents of the present invention are included in the scope that the present invention intends to protect.

Unless otherwise indicated, the raw materials and reagents used in the following examples are commercially available or may be prepared by known methods.

Example 1: Preparation of poly-L-lactic acid microspheres

10 g of poly-L-lactic acid (PLLA) (molecular weight 20000 Da) was dissolved in 150 mL of dichloromethane. The polymer solution was then added to 2000 mL of 1.0% (by mass) aqueous polyvinyl alcohol solution. After emulsified at 5000 rpm/min for 5 min, the mixture was further stirred at 1000 rpm for 6 h to remove dichloromethane, finally giving poly-L-lactic acid microspheres.

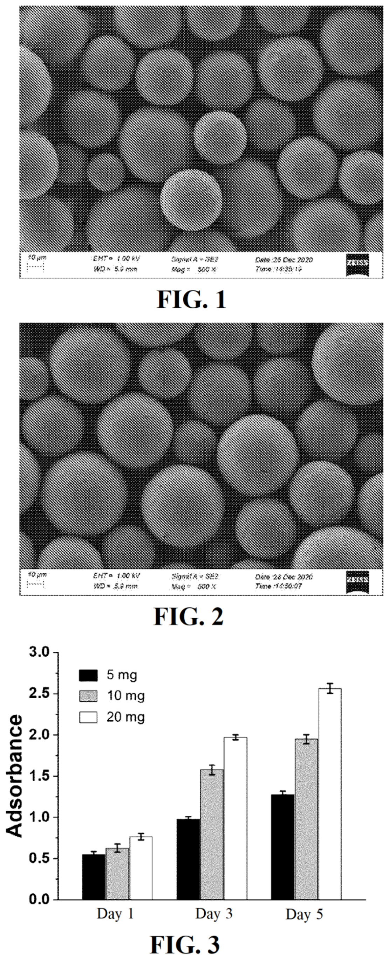

The resulting poly-L-lactic acid microspheres were observed using a scanning electron microscope and their particle size was calculated. The picture of the microspheres is shown in Fig. 1. As shown in Fig. 1, the microspheres have a particle size of 24.31-54.29 μm.

Example 2: Preparation of poly(lactic acid-glycolic acid) microspheres

10 g of poly(lactic acid-glycolic acid) (PLGA) (molecular weight 20000 Da) was dissolved in 150 mL of dichloromethane. The polymer solution was then added to 2000 mL of 0.5% (by mass) aqueous polyvinyl alcohol solution. After emulsified at 5000 rpm/min for 5 min, the mixture was further stirred at 1000 rpm for 6 h to remove dichloromethane, finally giving PLGA microspheres where the molar content of L-lactic acid is 0.0104 mol/g.

The resulting polylactic acid microspheres were observed using a scanning electron microscope and their particle size was calculated. The picture of the PLGA microspheres is shown in Fig. 2. As shown in Fig. 2, the microspheres have a particle size of 17.89-55.87 μm.

Example 3: Cellular experiments for promoting tendon growth

Tendon cells were extracted from the subscapularis muscle tendon of 2-week-old rabbits. The proliferation of rabbit tendon cells in PLLA microspheres was tested using the CCK-8 assay. First, 5 mg, 10 mg, and 20 mg of the polymer microspheres prepared in Example 1 were respectively added to the bottom of a 96-well plate, with 5 parallel wells for each sample. Then, 200 μL of cell suspension with a concentration of $4 \times 10^4$/mL was added to each sample well and placed in a cell culture incubator. After incubation in the dark for 1, 3, and 5 days, the cell culture medium in the 96-well plate was removed and placed in an empty 96-well plate, 20 μL of CCK-8 solution was added to each well, and after incubation in the dark for 4 hours, the absorbance at 450 nm was measured using a microplate reader. The proliferation results of rabbit tendon cells on PLLA microspheres of different concentrations were finally calculated. The results are shown in Fig. 3.

As shown in Fig. 3, the number of tendon cells gradually increases with the increase of PLLA microsphere concentration. Therefore, PLLA microspheres have a significant effect on promoting tendon cell proliferation, and their effect is significantly enhanced with increasing drug concentration. The inventors tested the effect of the polymer microspheres on ligament cells using the same method and obtained the same results, indicating that PLLA microspheres have a significant effect on promoting ligament cell proliferation.

Example 4: Effects of PLLA and PLGA microspheres on tendon growth in experimental animals

Experimental animals: C57BL mice, weighing 25±3 g (male and female in a 1:1 ratio), were divided into two groups of 10 mice each.
Experimental site: right Achilles tendon of mouse.
Experimental groups:

(1) 0.5 mg PLLA microspheres (Example 1);
(2) 0.5 mg PLGA microspheres (Example 2).

Experimental procedure: The anatomical location of the right Achilles tendon of the mouse was determined, the skin on the dorsal side of the distal tibia was disinfected with an alcohol swab, and injection was made into the Achilles tendon using a 26 G needle and a 1 mL sterile syringe (the angle between the needle and the Achilles tendon should be between 15-30° to avoid puncturing the tendon). The right side was designated as the experimental side (in situ injection of a solution of PLLA microspheres or PLGA microspheres in normal saline, only once; the dosage of L-lactic acid in the PLLA microsphere normal saline solution was 69.44 mmol/L, and the dosage of L-lactic acid in the PLGA microsphere normal saline solution was 52.08 mmol/L), while the left side was designated as the control side (injection of normal saline).

Three mice from each group were sacrificed at 7, 14 and 28 days respectively after surgery. The experimental and control sides of each mouse were selected for fixation and histopathological examination.

2. Test method:

1) The tissue was excised for observation, fixed, and stained with resorcinol fuchsin to observe the effect of microsphere injection on tissues at different sites.

2) Experimental and control sides were randomly selected for sectioning, the tissue diameter was measured under a 100x optical microscope, and the means values of the experimental and control sides were calculated using the following formula:

Mean = (3 measurements from animal ① + 3 measurements from animal ② + 3 measurements from animal ③) 30 / 9

3. Experimental results:

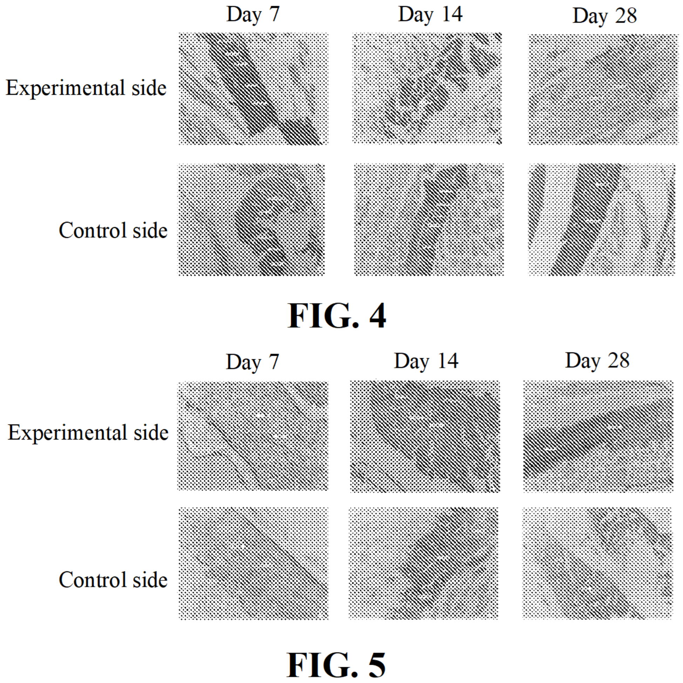

The experimental results are shown in Figs. 4 and 5. After injection of 0.5 mg PLLA microspheres or 0.5 mg PLGA microspheres, significant thickening of the tendon tissue on the experimental side was observed in both groups of experimental animals. As can be seen from the figure, the area of collagen fibers (red) on the experimental side was large on days 7, 14 and 28, indicating that the tendon was significantly thickened and the Achilles tendon diameter increased. Table 1 lists the average diameter of the Achilles tendon on the experimental side and the control side in the two groups.

The tendon tissue measurements under a 100x optical microscope are shown in Table 1. Compared with the control side, the differences in Achilles tendon diameter of the experimental side treated with 0.5 mg PLLA microspheres on days 14 and 28 were statistically significant (P<0.05), showing an increase in Achilles tendon diameter and demonstrating a significant promoting effect on tendon growth. Compared with the control side, the differences in Achilles tendon diameter of the experimental side treated with 0.5 mg PLGA microspheres on days 14 and 28 were statistically significant (P<0.05), showing an increase in Achilles tendon diameter and demonstrating a significant promoting effect on tendon growth.

These experimental results show that PLLA microspheres or PLGA microspheres have a significant promoting effect on the regeneration of animal tendon tissue. The inventor further employed the same method to test the effects of PLLA microspheres or PLGA microspheres on animal ligament tissues, and obtained the same effects. This indicates that PLLA microspheres or PLGA microspheres also have a significant promoting effect on the regeneration of animal ligament tissues.

Table 1. Measurements of injection of PLLA microspheres or PLGA microspheres as compared with the control group

| | Time point | Group | Mean $\pm$ SD ($\mu$m) | P value |
|---|---|---|---|---|
| 0.5 mg PLLA microspheres | 7 days | Experimental group | 1016.5±92.7 | 0.921 |
| | | Control group | 1000.5+246.7 | |
| | 14 days | Experimental group | 1198.4±129.7 | 0.027 |
| | | Control group | 877.4±98.1 | |
| | 28 days | Experimental group | 1444.9±176.1 | 0.002 |
| | | Control group | 1177±245.2 | |
| 0.5 mg PLGA microspheres | 7 days | Experimental group | 2321.9+655.9 | 0.119 |
| | | Control group | 1567.5+64.9 | |
| | 14 days | Experimental group | 2466.4±178.9 | 0.002 |
| | | Control group | 1289.4±216.7 | |
| | 28 days | Experimental group | 2582.8+84.9 | 0.017 |
| | | Control group | 1396.4±337.1 | |

Finally, it should be noted that the above embodiments are only used to illustrate the technical solutions of the present invention and are not intended to limit it. Although the present invention has been described in detail with reference to preferred embodiments, those skilled in the art should understand that modifications or equivalent substitutions can be made to the technical solutions of the present invention without departing from the spirit and scope of the technical solutions of the present invention, and all such modifications or substitutions should be covered within the scope of the claims of the present invention.

**Claims**

1.  Use of polylactic acid and a copolymer thereof in preparation of a drug for promoting tendon/ligament growth, wherein the molecular weight of the polylactic acid is 400-300000 Da, and preferably 5000-20000 Da.

2.  The use according to claim 1, wherein the polylactic acid is poly-L-lactic acid.

3.  The use according to claim 1, wherein the polylactic acid copolymer comprises one or more of a poly(lactic acid-glycolic acid) copolymer, a poly(lactic acid-ethylene glycol) copolymer, a poly(ethylene glycol-lactic acid-glycolic acid) copolymer, a polylactic acid-chitosan copolymer, and a lactide-caprolactone copolymer,
    preferably, the polylactic acid copolymer is a poly(lactic acid-glycolic acid) copolymer.

4.  The use according to any one of claims 1 to 3, where the polylactic acid and the copolymer thereof are present in the form of a formulation.

5.  The use according to claim 4, wherein the formulation includes microspheres, micelles, or gels.

6.  The use according to claim 4, wherein the formulation further comprises an excipient, and the excipient includes at least one of a stabilizer, a filler, a binder, a surfactant and a lubricant.

7.  The use according to claim 6, wherein the surfactant includes one or more of polyethylene glycol, sodium dodecyl sulfonate, Tween, and Span; the stabilizer includes one or two of carboxymethyl cellulose and mannitol; the filler includes one or more of lactose, mannitol, cyclodextrin, and sorbitol; the binder includes one or more of hydroxypropyl cellulose, methyl cellulose, and polyvinylpyrrolidone; and the lubricant includes one or more of magnesium stearate, calcium stearate, and stearic acid.

8.  The use according to claim 4, wherein the formulation further comprises a first active ingredient, and the first active ingredient has the effect of reducing swelling and relieving pain.

9.  The use according to claim 8, wherein the first active ingredient includes celecoxib, loxoprofen sodium, phenylbutazone, ibuprofen, and etoricoxib.

10. The use according to claim 7, wherein during at least one administration of the formulation, the dosage of L-lactic acid in poly-L-lactic acid and copolymers thereof is 8-3000 mmol/L, preferably 250-750 mmol/L.

11. The use according to claim 5, wherein the particle size of the microspheres is in the range of 17 $\mu$m to 60$\mu$m.

12. The use according to claim 7, wherein the formulation is an injection, and the dosage of poly-L-lactic acid or copolymers thereof based on L-lactic acid in the injection is 8-3000 mmol/L, preferably 250-750 mmol/L or 50-100 mmol/L.

**FIG. 1**

**FIG. 2**

**FIG. 3**

| Day 7 | Day 14 | Day 28 |

Experimental side

Control side

**FIG. 4**

| Day 7 | Day 14 | Day 28 |

Experimental side

Control side

**FIG. 5**

## INTERNATIONAL SEARCH REPORT

| International application No. |
| --- |
| **PCT/CN2024/103598** |

| A. | CLASSIFICATION OF SUBJECT MATTER |
| --- | --- |

A61K47/34(2017.01)i; A61L17/10(2006.01)i; A61L27/58(2006.01)i; A61P41/00(2006.01)i

According to International Patent Classification (IPC) or to both national classification and IPC

| B. | FIELDS SEARCHED |
| --- | --- |

Minimum documentation searched (classification system followed by classification symbols)

IPC: A61K, A61L, A61P

Documentation searched other than minimum documentation to the extent that such documents are included in the fields searched

Electronic data base consulted during the international search (name of data base and, where practicable, search terms used)

CNTXT: WPABSC; ENTXTC; DWPI; VEN; CNKI; 万方, WANFANG; 百度学术, BAIDU SCHOLAR, Science Direct: 圣博玛生物材料, 聚乳酸, PLA, 聚乳酸羟基乙酸, PLGA, 聚乳酸-羟基乙酸, 聚乙二醇, 壳聚糖, 丙交酯己内酯, Poly lactic acid, poly lactic-glycolic acid, polyethylene glycol, chitosan, polylactide caprolactone

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 115607740 A (BEIJING DEYI DAMEI MEDICAL TECH. CO., LTD.) 17 January 2023 (2023-01-17) abstract, claim 1, and description, paragraph 41 | 1-12 |
| X | US 6840962 B1 (MASSACHUSETTS INSTITUTE OF TECHNOLOGY et al.) 11 January 2005 (2005-01-11) claims 1 and 3-5, and description, column 4, lines 20-25 and 45-52 | 1-12 |
| X | CN 104254351 A (TRB CHEMEDICA INTERNATIONAL SA) 31 December 2014 (2014-12-31) claims 1 and 13-14 | 1-12 |
| A | CN 1857235 A (CHENGDU ORGANIC CHEMICALS CO., LTD., CHINESE ACADEMY OF SCIENCES) 08 November 2006 (2006-11-08) claims 1-7 | 1-12 |
| A | CN 101400717 A (COLOPLAST AS) 01 April 2009 (2009-04-01) claims 8 and 23-24 | 1-12 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **04 September 2024** | **12 September 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/103598** |

| C. | DOCUMENTS CONSIDERED TO BE RELEVANT | |
|---|---|---|
| Category* | Citation of document, with indication, where appropriate, of the relevant passages | Relevant to claim No. |
| A | WO 2016122009 A1 (METABIOMED CO., LTD.) 04 August 2016 (2016-08-04)<br>claims 1-7 | 1-12 |

Form PCT/ISA/210 (second sheet) (July 2022)

## INTERNATIONAL SEARCH REPORT
### Information on patent family members

International application No.

**PCT/CN2024/103598**

| Patent document cited in search report | | | Publication date (day/month/year) | Patent family member(s) | | | Publication date (day/month/year) |
|---|---|---|---|---|---|---|---|
| CN | 115607740 | A | 17 January 2023 | None | | | |
| US | 6840962 | B1 | 11 January 2005 | US | 2007005139 | A1 | 04 January 2007 |
| | | | | US | 7914579 | B2 | 29 March 2011 |
| | | | | US | 6123727 | A | 26 September 2000 |
| | | | | US | 2005060033 | A1 | 17 March 2005 |
| CN | 104254351 | A | 31 December 2014 | TR | 201909420 | T4 | 22 July 2019 |
| | | | | SI | 2827914 | T1 | 30 August 2019 |
| | | | | BR | 112014023404 | B1 | 05 November 2019 |
| | | | | CA | 2864050 | A1 | 26 September 2013 |
| | | | | RS | 58902 | B1 | 30 August 2019 |
| | | | | EP | 2827914 | A1 | 28 January 2015 |
| | | | | EP | 2827914 | B1 | 08 May 2019 |
| | | | | ES | 2733313 | T3 | 28 November 2019 |
| | | | | HUE | 044160 | T2 | 28 October 2019 |
| | | | | PL | 2827914 | T3 | 30 September 2019 |
| | | | | KR | 20140138867 | A | 04 December 2014 |
| | | | | DK | 2827914 | T3 | 15 July 2019 |
| | | | | RU | 2014141701 | A | 20 May 2016 |
| | | | | RU | 2629809 | C2 | 04 September 2017 |
| | | | | MX | 2014011342 | A | 22 January 2015 |
| | | | | MX | 368000 | B | 13 September 2019 |
| | | | | LT | 2827914 | T | 25 July 2019 |
| | | | | HRP | 20191077 | T1 | 20 September 2019 |
| | | | | PT | 2827914 | T | 04 July 2019 |
| | | | | CY | 1121785 | T1 | 31 July 2020 |
| | | | | JP | 2015512282 | A | 27 April 2015 |
| | | | | US | 2015045887 | A1 | 12 February 2015 |
| | | | | US | 9649190 | B2 | 16 May 2017 |
| | | | | WO | 2013139955 | A1 | 26 September 2013 |
| CN | 1857235 | A | 08 November 2006 | None | | | |
| CN | 101400717 | A | 01 April 2009 | CA | 2645153 | A1 | 13 September 2007 |
| | | | | ES | 2338820 | T3 | 12 May 2010 |
| | | | | US | 2009047322 | A1 | 19 February 2009 |
| | | | | US | 8877223 | B2 | 04 November 2014 |
| | | | | PT | 1996643 | E | 15 April 2010 |
| | | | | ATE | 458773 | T1 | 15 March 2010 |
| | | | | DE | 602007004955 | D1 | 08 April 2010 |
| | | | | WO | 2007101443 | A1 | 13 September 2007 |
| | | | | WO | 2007101443 | A8 | 08 November 2007 |
| | | | | DK | 1996643 | T3 | 14 June 2010 |
| WO | 2016122009 | A1 | 04 August 2016 | KR | 20160091676 | A | 03 August 2016 |
| | | | | KR | 102331359 | B1 | 26 November 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310840075X